# EUROPEAN PATENT SPECIFICATION

(11) **EP 2 826 429 B1**
(45) Date of publication and mention of the grant of the patent: **14.09.2016**
(21) Application number: 13177279.0
(22) Date of filing: 19.07.2013
(51) Int. Cl.: A61B 17/70

(54) **Polyaxial bone anchoring device**
Polyaxiale Knochenverankerungsvorrichtung
Dispositif d'ancrage d'os polyaxial

(43) Date of publication of application: 21.01.2015
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE)
(74) Representative: Prüfer & Partner mbB Patentanwälte · Rechtsanwälte

(56) References cited:
- EP-A1- 1 795 134
- EP-A1- 2 174 608
- DE-A1-102010 028 423
- US-A1- 2008 045 953
- US-A1- 2012 143 266

## Description

The invention relates to a polyaxial bone anchoring device that includes an anchoring element with a head and a shank and a receiving part comprising a seat for pivotably receiving the head and a recess forming a channel to receive a single rod therein. The polyaxial bone anchoring device further comprises a first clamping part for supporting the single rod in the recess from its lower side and a second clamping part for acting onto the upper side of the single rod. The first clamping part and the second clamping part both extend laterally into the channel provided by the recess. The polyaxial bone anchoring device further comprises a locking device that is configured to exert pressure on the second clamping part. Such a polyaxial bone anchoring device is particularly suitable in connection with rods having a surface that is sensitive to corrosion, abrasion and wear and in connection with curved rods.

A spinal stabilization system with the features as defined in the preamble of claim 1 is known from EP 1 795 134 A1.

A polyaxial bone anchoring device wherein the rod is clamped between a pressure element having a recess for the rod and a set screw is known from US 2003/0100896 A1 and EP 2 174 608 A1.

Various other solutions for clamping a specific type of rod for improving the fixation of such rods are known. For example, US 2008/0086132 A1 describes a polyaxial bone anchoring device for tubular rods. A filling piece is arranged between a locking element and the rod, wherein the filling piece comprises a rod contacting surface which contacts a portion of the rod and wherein the shape of the rod contacting surface is adapted to the shape of the portion of the rod.

Another bone anchoring device according to US 2007/0093820 A1 also describes a filling piece between the rod and a clamping element, such as a threaded nut, wherein the filling piece comprises transverse ribs to clamp a rod made of an elastomer material.

US 7,338,491 B2 describes a locking mechanism configured to engage and lock a relative position of a bone fixation device and a relative position of a stabilization device with a locking element that comprises a rectangular shape and is longer than the width of the seat such that when the locking element and the seat are engaged the locking element protrudes from the side portion.

In particular when using rods made fully or partially of Nitinol a reduced contact area for clamping may lead to increased local forces and an increased notch effect or even plastic deformation of the rod or the clamping part.

There is still a need for a polyaxial bone anchoring device with an improved rod clamping design.

It is the object of the invention to provide a spiral stabilization system that has an improved rod clamping design so that it is suitable for a variety of rods.

The object is solved by a spiral stabilization system according to claim 1. Further developments are given in the dependent claims.

The spiral stabilization system has a first and a second clamping part that extend into the channel of the receiving part that accommodates the single rod. Hence, a greater surface for supporting and clamping the single rod is provided. By the greater clamping surface, a notch effect is reduced and there is no or only little plastic deformation of the parts.

Furthermore, the clamping pressure is distributed over a greater area compared to clamping the single rod with only a set screw.

A possible damage of the rod surface that may result in corrosion and/or nickel release in the case of Nitinol rods can be prevented.

The first and the second clamping part can be slightly flexible so that they adapt to the surface of the single rod when they are pressed against the single rod.

Multiple contact areas provided by the first and the second clamping part allow to use also curved rods, for example curved MIS rods (rods for minimally invasive surgery).
Further features and advantages of the invention will become apparent from the description of embodiments by means of the accompanying drawings.

In the drawings:
- Fig. 1: shows a perspective exploded view of a polyaxial bone anchoring device with a straight rod according to a first embodiment.
- Fig. 2: shows a perspective view of the polyaxial bone anchoring device according to Fig. 1 in an assembled state with the rod clamped.
- Fig. 3: shows a cross-sectional view of the polyaxial bone anchoring device according to Fig. 2, wherein the section is taken in a plane through the central axis of the receiver and perpendicular to the rod axis.
- Fig. 4a: shows a cross-sectional view of the polyaxial bone anchoring device in an assembled state according to Fig. 2, wherein the section is taken in a plane containing the central axis of the receiving part and the rod axis.
- Fig. 4b: shows a cross-sectional view of the polyaxial bone anchoring device as in Fig. 2 with a curved rod.
- Fig. 5: shows a perspective view from the top of a first clamping part of the polyaxial bone anchoring device according to the first embodiment.
- Fig. 6: shows a perspective view from the bottom of the clamping part of Fig. 5.
- Fig. 7: shows a top view of the clamping part of Fig. 5.
- Fig. 8: shows a cross-sectional view of the clamping part along line D-D in Fig. 7.
- Fig. 9: shows a side view of the clamping part of Fig. 5.
- Fig. 10: shows a perspective view from the top of an second clamping part of the polyaxial bone anchoring device according to the first embodiment.
- Fig. 11: shows a perspective view from the bottom of the clamping part according to Fig. 10.
- Fig. 12: shows a top view of the clamping part according to Fig. 10.
- Fig. 13: shows a cross-sectional view of the clamping part according to line A-A in Fig. 12.
- Fig. 14: shows a side view of the clamping part shown in Fig. 10.
- Fig. 15: shows a perspective exploded view of a polyaxial bone anchoring device with a rod according to a second embodiment.
- Fig. 16: shows a perspective view of the polyaxial bone anchoring device according to Fig. 15 in an assembled state with the rod clamped.
- Fig. 17: shows a cross-sectional view of the polyaxial bone anchoring device according to Fig. 16, wherein the section is taken in a plane through the central axis of the receiving part and perpendicular to the rod axis.
- Fig. 18: shows a cross-sectional view of the polyaxial bone anchoring device according to Fig. 16, wherein the section is taken in a plane through the central axis of the receiving part and contains the rod axis.
- Fig. 19: shows a perspective view from the top of a first clamping part of the polyaxial bone anchoring device according to the second embodiment.
- Fig. 20: shows a perspective view from the bottom of the clamping part of Fig. 19.
- Fig. 21: shows a top view of the clamping part of Fig. 19.
- Fig. 22: shows a cross-sectional view of the clamping part along line C-C in Fig. 21.
- Fig. 23: shows a side view of the clamping part of Fig. 19.
- Fig. 24: shows a perspective view from the top of a second clamping part according to polyaxial bone anchoring device of the second embodiment.
- Fig. 25: shows a perspective view from the bottom of the upper clamping part shown in Fig. 24.
- Fig. 26: shows a top view of the clamping part of Fig. 24.
- Fig. 27: shows a cross-sectional view of the clamping part along line B-B in Fig. 26.
- Fig. 28: shows a side view of the clamping part of Fig. 24.
- Fig. 29: shows a perspective exploded view of a polyaxial bone anchoring device with a rod according to a third embodiment.
- Fig. 30: shows a cross-sectional view of the polyaxial bone anchoring device according to Fig. 29, wherein the section is taken in a plane through the central axis of the receiving part and perpendicular to the rod axis.

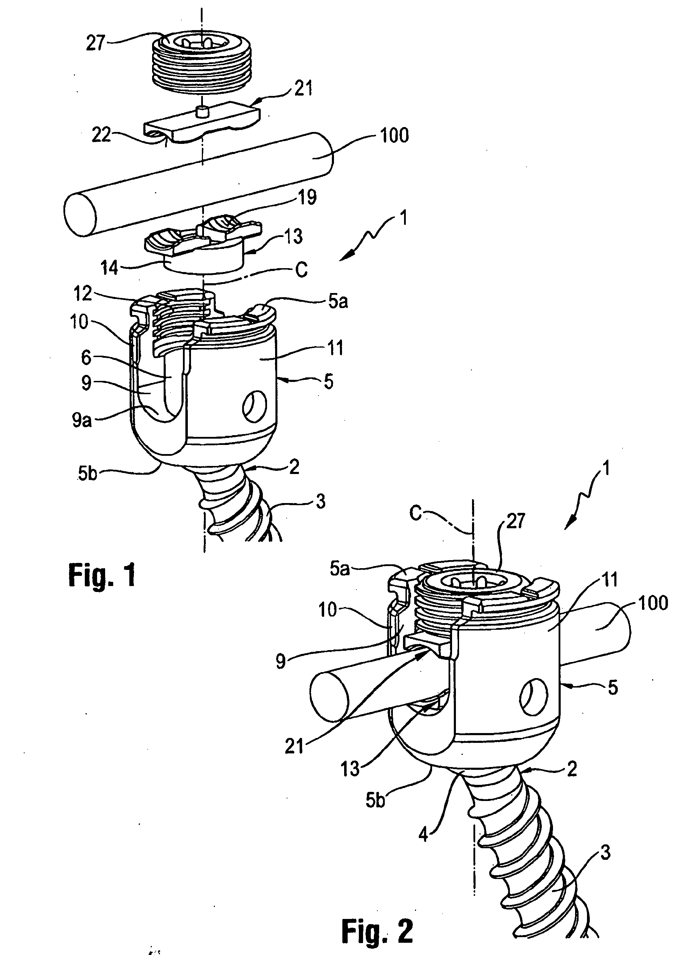

Referring to Figs. 1 to 4, the polyaxial bone anchoring device 1 according to a first embodiment includes a bone anchoring element 2 in the form of a bone screw having a shank 3 with a threaded portion and a tip (not shown) and a head 4. In the example shown, the head 4 has a spherical segment shape. A recess 4a for engagement with a tool is provided at the free end of the head 4.

The polyaxial bone anchoring device further comprises a receiving part 5 that has a first end 5a and an opposite second end 5b, a central axis C going through the planes defined by the first end 5a and the second end 5b respectively, and a coaxial bore 6 extending from the first end 5a to a distance from the second end 5b. At the second end 5b, an opening 7 is provided, a diameter of which is smaller than a diameter of the bore 6. A spherically-shaped section 8 is provided adjacent to the opening 7 that forms a seat for the head 4. The section that forms the seat can have other shapes, for example, a conical shape, that allow to pivotably hold the head 4 therein.

The receiving part 5 comprises a substantially U-shaped recess 9 that starts at the first end 5a and extends to a distance from the second end 5b for receiving a rod 100. The substantially U-shaped recess has a bottom 9a. By means of the U-shaped recess 9, two free legs 10, 11 are formed. On the legs 10, 11 an internal thread 12 is provided.

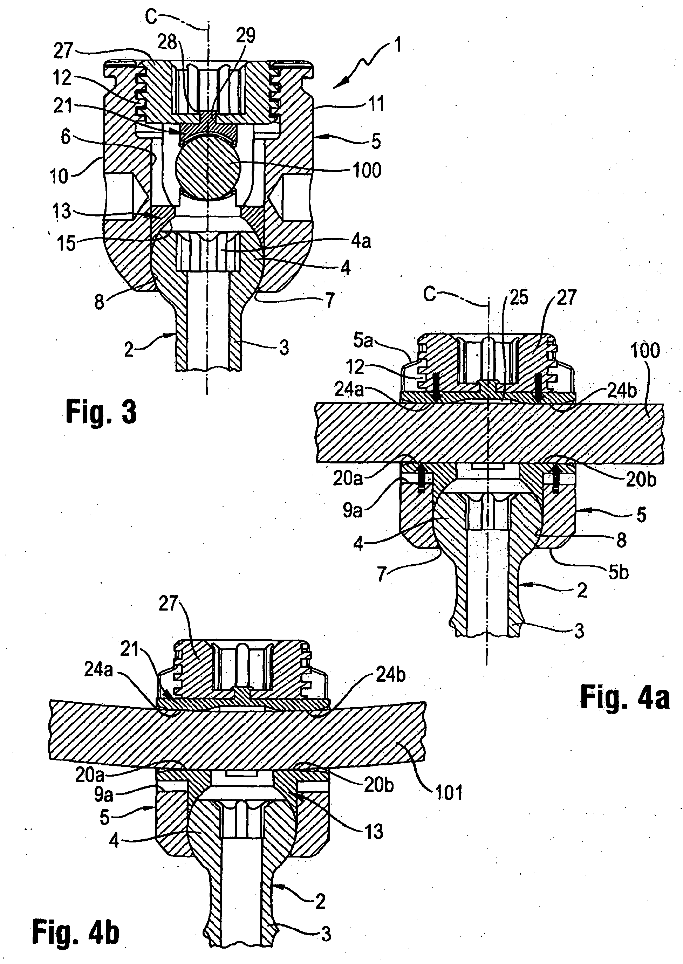

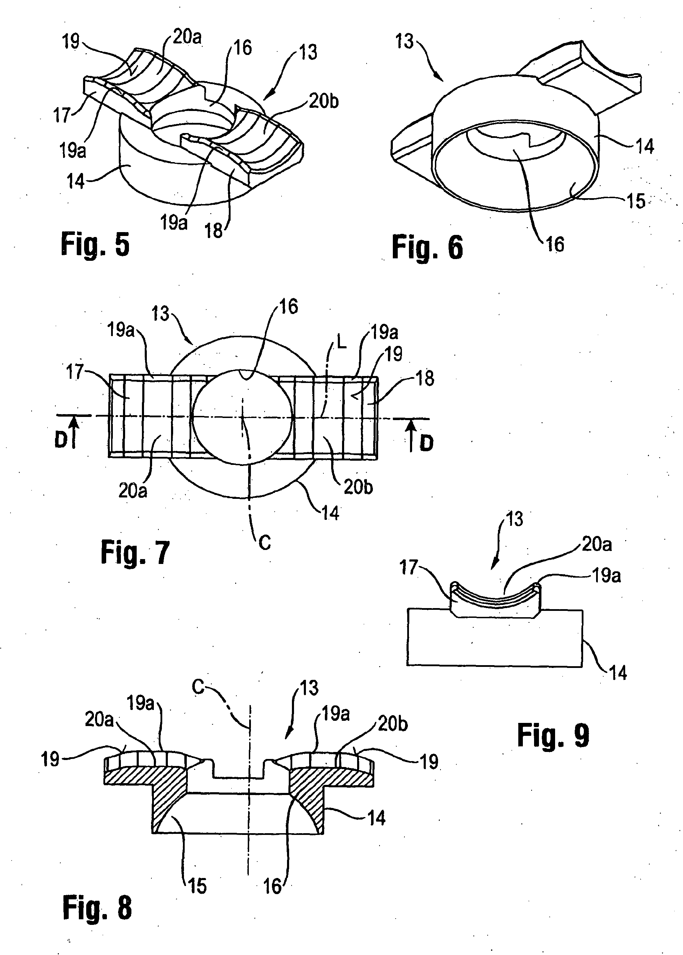

Referring now further to Figs. 5 to 9, the polyaxial bone anchoring device 1 further comprises a first clamping part 13 supporting the rod 100 from the lower side of the rod. In the embodiment shown, the first clamping part 13 is a monolithic piece. It comprises a first substantially cylindrical portion 14 with an outer diameter that is only slightly smaller than the inner diameter of the bore 6 to allow the first clamping part 13 to be moved in the axial direction once it is inserted in the bore 6. On its lower side facing towards the second end 5b of the receiving part, the first clamping part 13 comprises a spherical segment-shaped recess 15 the radius of which correspond substantially to radius of the head 4 of the anchoring element 2. The height of the cylindrical portion 14 in axial direction is, as can be seen in particular in Fig. 4, such that when the first clamping part 13 rests on the head 4 of the anchoring element 2, the cylindrical portion 14 extends above the bottom 9a of the substantially U-shaped recess 9 of the receiving part 5.

The first clamping part 13 further comprises a coaxial bore 16 that allows access to the head 4 of the anchoring element with a tool when the anchoring element is held in the receiving part 5. On its side opposite to the spherical recess 15, the first clamping part 13 comprises two laterally extending arms 17, 18, that are offset from each other by 180° and extend from the bore 16 in a direction perpendicular to the bore axis beyond the outer surface of the first cylindrical portion 14. The arms 17, 18 have a substantially rectangular outer contour with a circular segment portion facing the coaxial bore 16 and having a corresponding diameter. An upper surface of the arms 17, 18 forms a rod supporting surface 19 that is substantially groove-shaped with a longitudinal axis L of the groove being perpendicular to the central axis C. As can be seen in particular in Figs. 5 and 9, the shape of the groove is substantially a cylinder-segment with an inner diameter that is only slightly larger than corresponding diameter of a cylinder segment of the rod 100, so that when the rod 100 is placed on the rod supporting surface 19 it fits into the groove as can be seen in Fig. 3.

Referring to Fig. 7 and 8, the rod supporting surface 19 comprises along the longitudinal axis L portions with different depth. Each arm has a portion 20a, 20b with a smallest depth, whereby two bulges 20a, 20b are formed that each decline smoothly in a longitudinal direction towards the coaxial bore 16 on one side and towards the outer free end of the arms 17, 18 on the other side. An upper surface of the bulges 20a, 20b may be even. The steepness of the declining portions to the right and left of the bulges 20a, 20b in the longitudinal direction may be the same or may be different. For example, the steepness may be greater towards the outer free end of the arms 17, 18 than towards the coaxial hole 16. A rim 19a of the rod supporting surface 19 varies in height corresponding to the portions and has a highest position at the bulges 20a, 20b. The bulges 20a, 20b have a width in the longitudinal direction that provides a contact with the rod that is greater than a line contact.

The arms 17, 18 have such a size that they extend substantially up to the end of the channel formed by the U-shaped recess 9 in the receiving part. As can be seen in Fig. 4, the free end of the arms 17, 18 is substantially flush with the outer surface of the receiving part 5. The width of the arms is only slightly smaller than the width of the U-shaped recess 9 of the receiving part 5, so that the arms are guided in the recess 9.

The rod supporting surface 19 provides an enlarged contact area for the rod compared to conventional pressure elements the size of which is confined to the size of the bore 6. By means of the bulges 20a, 20b two contact areas that are spaced apart from each other at a distance greater than the diameter of the bore 6 are provided. Also, the rounded design of the contact areas enlarges the possibilities of contact between the rod 100 and the first clamping part 13. By means of this, it is possible to clamp also rods that have curvature, such as curved MIS rods.

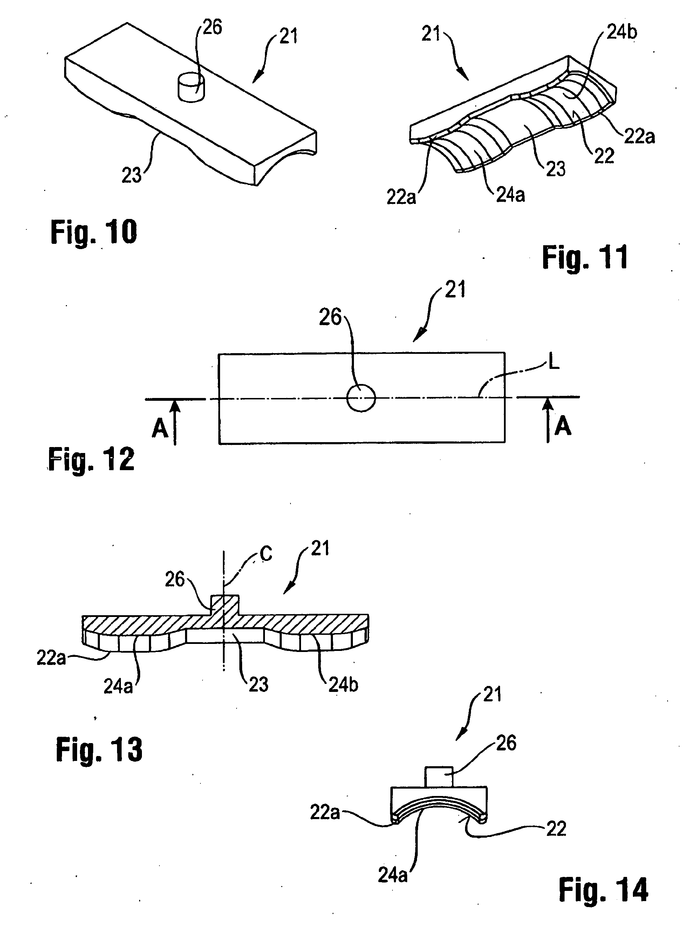

Referring now additionally to Figs. 10 to 14, the polyaxial bone anchoring device also comprises a second clamping part 21. As can be seen in Fig. 12, the second clamping part 21 has a substantially rectangular contour. A length of the second clamping part corresponds substantially to a length of the channel formed by the U-shaped recess 9 of the receiving part. A width of the second clamping part 21 is only slightly smaller than a width of the U-shaped recess 9 of the receiving part 5 so that the second clamping part 21 may be inserted into the U-shaped recess and it is guided therein.

A lower side of the second clamping part 21 that faces the rod 100 when the second clamping part 21 is in the receiving part 5, is substantially groove-shaped with a width of the groove being substantially the same as a width of the groove of the first clamping part 13. The surface of the groove forms a rod facing surface 22 that is configured to come into contact with the rod and to exert pressure onto the rod 100 with at least a portion of the surface. The rod facing surface 22 comprises a substantially flat central portion 23 and two bulges 24a, 24b on each side of the central portion 23. Similar to the first clamping part 13 the bulges 24a, 24b form shallow portions of the groove in the longitudinal direction. The top of the bulges 24a, 24b may be even. The bulges 24a, 24b decline smoothly towards the free ends and towards the center portion of the second clamping part 21 with the same or with different steepness. A rim 22a of the rod facing surface 22 varies in height corresponding to the portions 22, 24a, 24b of the rod facing surface and has a highest position at the position of the bulges 24a, 24b.

A distance between the bulges 24a and 24b of the second clamping part 21 can be the same as the distance between the bulges of the first clamping part 13. The distance may also be different.

As can be seen in Fig. 4a and 4b, because the rim 22a of the rod facing surface 22 is lowered in the central portion 23, a gap 25 between the rod 100 and the rod contacting surface 22a is formed.

On its side facing away from the rod facing surface 22, the second clamping part 21 comprises a projection 26 that is cylindrical and serves for rotationally engage a locking screw 27 as shown in Figs. 3 and 4. The second clamping part 21 is rotatably coupled to the locking screw 27 that cooperates with the internal thread 12 of the legs 10, 11 of the receiving part 5. As depicted in Fig. 3, the second clamping part 21 may have a rim 28 at the end of the cylindrical projection 26 that holds the second clamping part 21 in a bore 29 at the lower side of the locking screw 27. An upper side of the second clamping part 21 that faces towards the locking screw 27 is substantially even.

All parts of the bone anchoring device are made from a body compatible material, such as a body compatible metal or metal alloy or a body compatible plastic material. Examples for such metals or metal alloys are titanium, stainless steel, titanium alloys, such as, for example Nitinol. An example for a body compatible plastic is PEEK (Polyetheretherketone).

At least two bone anchoring devices and a rod form a spinal stabilization system. The rods to be used are preferably rods that have a more sensitive surface or rods that may cause problems if used with conventional bone anchoring devices. In particular, the polyaxial bone anchoring device can be used with Nitinol rods. Another type of rods that can be used are curved rods, in particular curved MIS rods.

In use, the first clamping part 13 may be pre-assembled with the receiving part 5 and the anchoring element 2. The first clamping part 13 can be inserted by orientating it such that the arms 17, 18 extend into the U-shaped recess 9. The second clamping part 21 may be pre-assembled with the locking screw 27. First, two polyaxial bone anchoring devices consisting of the pre-assembled receiving part 5 with anchoring element 2 and first clamping part 13 are inserted into two bone parts or two vertebrae, respectively. Thereafter, the rod is inverted into the U-shaped recess 9 of the receiving part 5 until it rests with its lower side on the bulges 20a, 20b of the rod supporting surface 19. Then, the pre-assembled locking screw 27 with the first clamping part 21 is inserted into the receiving part until the rod facing surface 22 contacts the rod 100 at the contact areas provided by the bulges 24a, 24b. Due to the rotatable connection, the locking screw 27 can be tightened while the second clamping part 21 engages the rod 100. When the locking screw 27 is tightened, pressure is exerted via the second clamping part 21 onto the rod 100 and from the rod 100 onto the first clamping part 13. The first clamping part 13 also exerts pressure onto the head 4. The pressure exerted onto the rod 100 is distributed over a larger area compared to conventional devices. Furthermore, the pressure is exerted via multiple contact areas, in particular, via four contact areas as shown in Figs. 4a and b. The contact areas are provided by the uppermost portion of the bulges 20a, 20b, 24a, 24b. In the embodiment shown, the contact areas of the rod supporting surface 19 and the rod facing surface 22 lie at the same longitudinal positions along the rod axis. Final tightening of the locking screw 27 also locks the head 4 in an angular position through the pressure exerted onto it by the first clamping part 13.

Due to the enlarged contact area, a notch effect that may lead to corrosion and abrasion and the setting free of nickel particles may be reduced or even prevented.

While Fig. 4a shows the bone anchoring device with a straight rod 100, Fig. 4b shows the bone anchoring device with a curved rod 101. The smooth shape of the bulges 20a, 20b, 24a, 24b allows the clamping parts to adapt the contact area to the shape of the rod.

It shall be understood that in a modified first embodiment, more than two contact areas on each of the clamping parts 13,21 may be provided. Also, the contact areas of the first clamping part 13 and the contact areas of the second clamping part 21 may have a different distance from each other so that the rod is clamped at different positions in a longitudinal direction from below and from above.

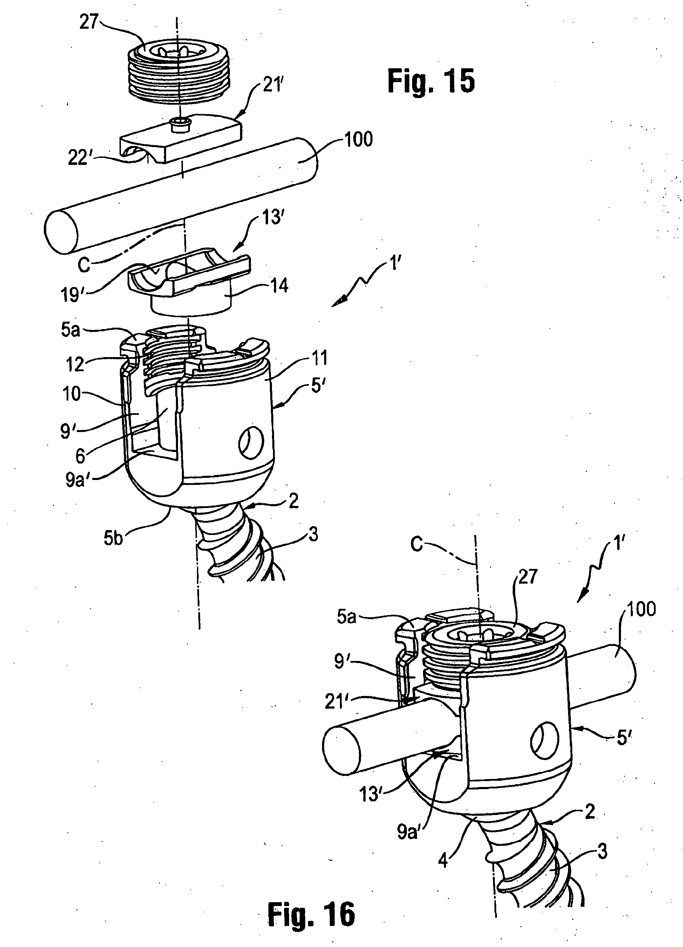

A second embodiment of the polyaxial bone anchoring device will be described with reference to Figs. 15 to 18. The polyaxial bone anchoring device 1' differs form the polyaxial bone anchoring device according to the first embodiment mainly by the design of the first and the second clamping part. All other parts are the same or identical to the first embodiment and the parts and portions that are the same or highly similar to that of the first embodiment have the same references numerals and the description thereof will not be repeated.

The receiving part 5' has in this embodiment a recess 9' for receiving the rod with a substantially rectangular contour and a flat bottom 9a'.

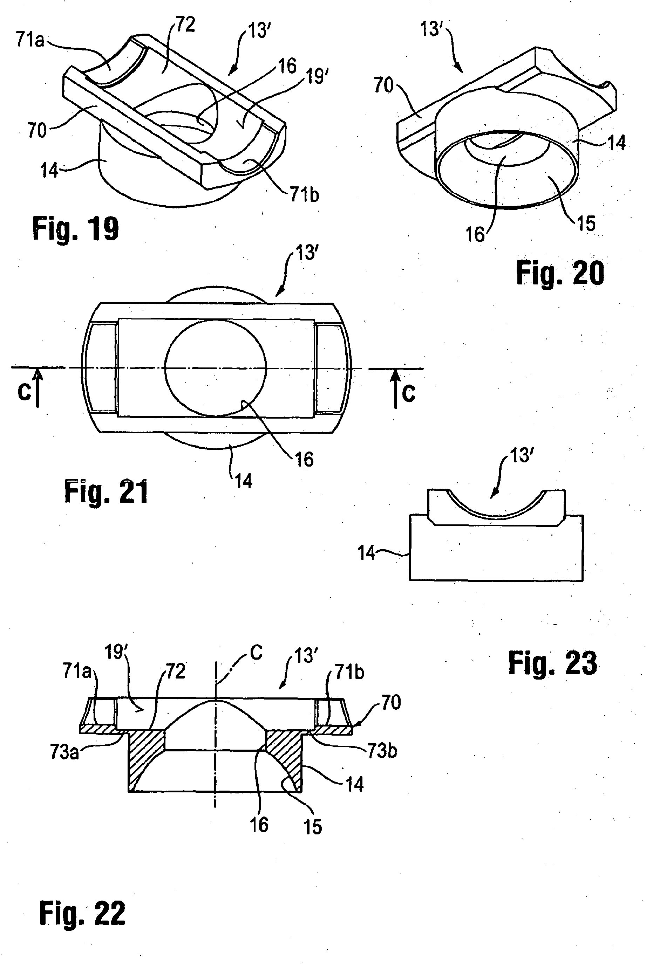

The first clamping part 13' comprises the cylindrical portion 14 with the spherically-shaped recess 15 at the underside and the coaxial bore 16. It differs only in the rod supporting surface 19'. The rod supporting surface 19' is formed on a groove-like portion 70 that is provided at the side of the cylindrical portion 14 opposite to the spherically-shaped recess 15. The groove-like portion 70 has a substantially rectangular contour with rounded short sides, as shown in Fig. 21. A total length in the longitudinal direction along the longitudinal axis L of the groove-like portion 70 corresponds substantially to the axial length of the recess 9' in the receiving part 5' measured perpendicular to the central axis C. A width of the groove portion 70 is only slightly smaller than a width of the recess 9' in the receiving part 5' and allows to place the groove-like portion 70 between the legs 10, 11 that are separated by the recess 9'.

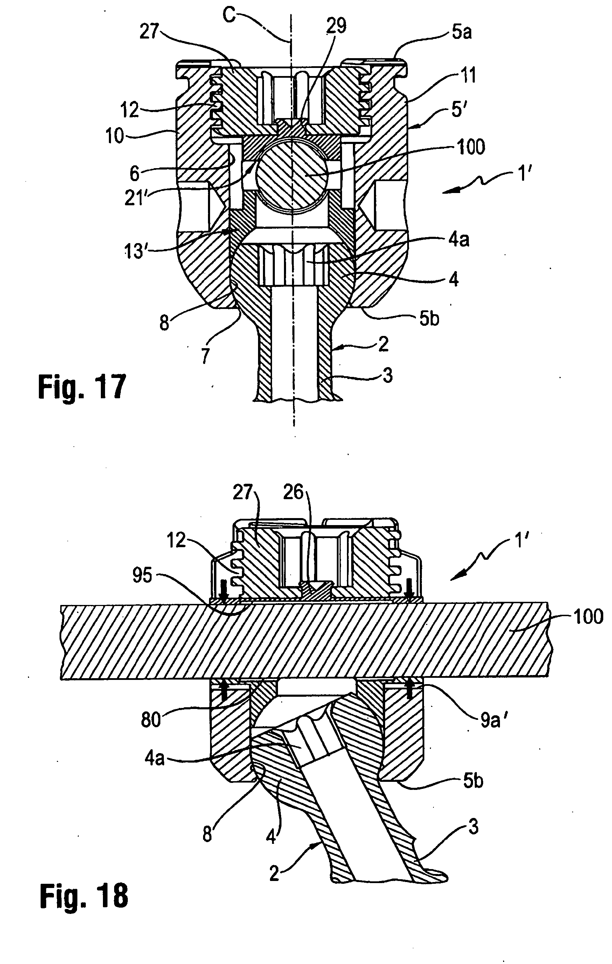

The rod supporting surface 19' comprises on each outer end of the groove-like portion 70 a shallow part 71a, 71b and a deeper part 72 there between. Hence, the shallow parts form slight prominences 71a, 71b that may have an even upper surface. Between the prominences 71a, 71b and the cylindrical portion 14, the groove-like portion 70 has thinner bottom portions 73a, 73b as can be seen in Fig. 22. This renders the groove-like portion 70 slightly flexible. As can be seen in particular in Fig. 18, there is a gap 80 between the rod supporting surface 19' in the shallower region 72 and the rod 100 when it rests on the prominences 71a, 71b. The gap 80 allows the groove-like portion 70 to adapt to the surface of the rod when pressure is exerted via the rod onto the rod supporting surface 19' of the first clamping part 13'.

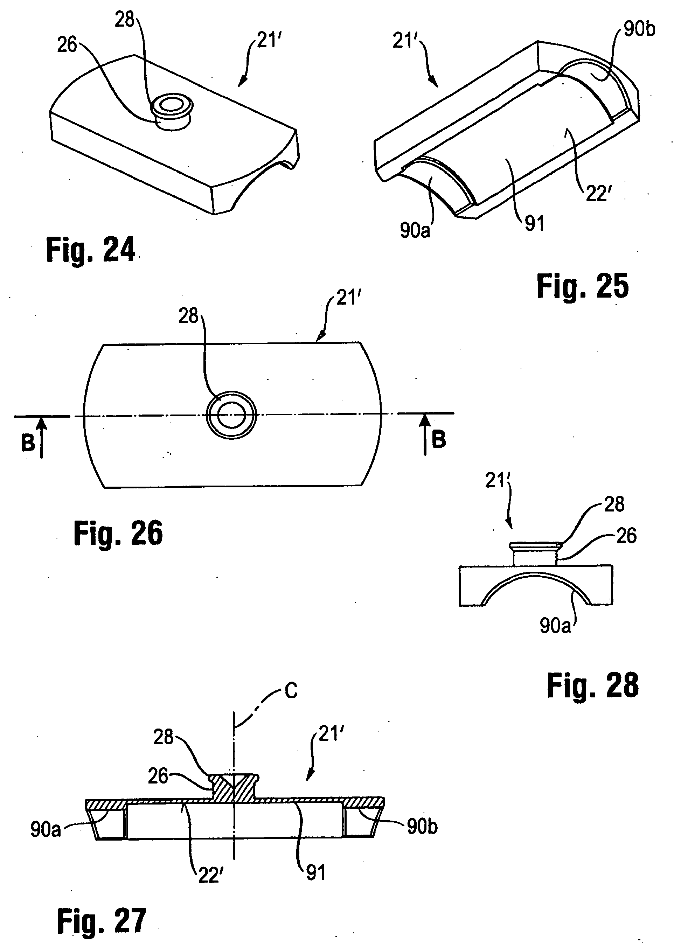

Referring further to Figs. 24 to 27, the second clamping part 21' has an outer contour corresponding to that of the groove-like portion 70 of the first clamping part 13'. The second clamping part 21' comprises a rod facing surface 22' that has a complementary shape to the rod supporting surface 19' of the first clamping part 13'. That means, it has a groove-like shape with prominences 90a, 90b at either end of the second clamping part 21' that form rod contact areas and a cylinder-segment shaped recess 91 between the prominences 90a, 90b.

The second clamping part 21' is rotatably connected to the locking screw 27 as in the first embodiment. An upper surface of the second clamping part 21' that faces the locking element 27 is substantially even in order to allow to transfer the pressure from the locking element 27 evenly onto the second clamping part 21'.

As can be seen in Fig. 18, the recess 91 leads to a gap 95 between the rod 100 and the rod facing surface 22'. This gap 95 renders the second clamping part 21' slightly flexible and allows to adapt the rod facing surface 22' to the surface of the rod 100.

In use, when the locking screw 27 is tightened, the pressure exerted by the locking screw 27 is transferred via the second clamping part 21' and the rod 100 onto the first clamping part 13'. Preferably the rod contacting areas are positioned at the same locations in an axial direction along the rod axis on the lower side and the upper side of the rod 100. Because the groove-like portion 70 of the first clamping part 13' and the second clamping part 21' are slightly flexible, they can adapt to the surface of the rod. Hence, the clamping area can be enlarged resulting in an improved clamping without the risk of notch effects and plastic deformation, as well as of abrasion and corrosion. When the rod presses onto the rod supporting surface 19', the first clamping part 13' exerts a pressure onto the head 4 that locks the head 4 in a specific angular position.

It shall be understood that the second embodiment may also used with curved rods, because of the ability of the clamping parts to adapt to a surface of the rod. Furthermore, the position and number of the prominences is not limited to the position and number of the prominences as shown in this specific embodiment. There may be more than two contact areas. The contact areas may also be positioned away from the outer ends.

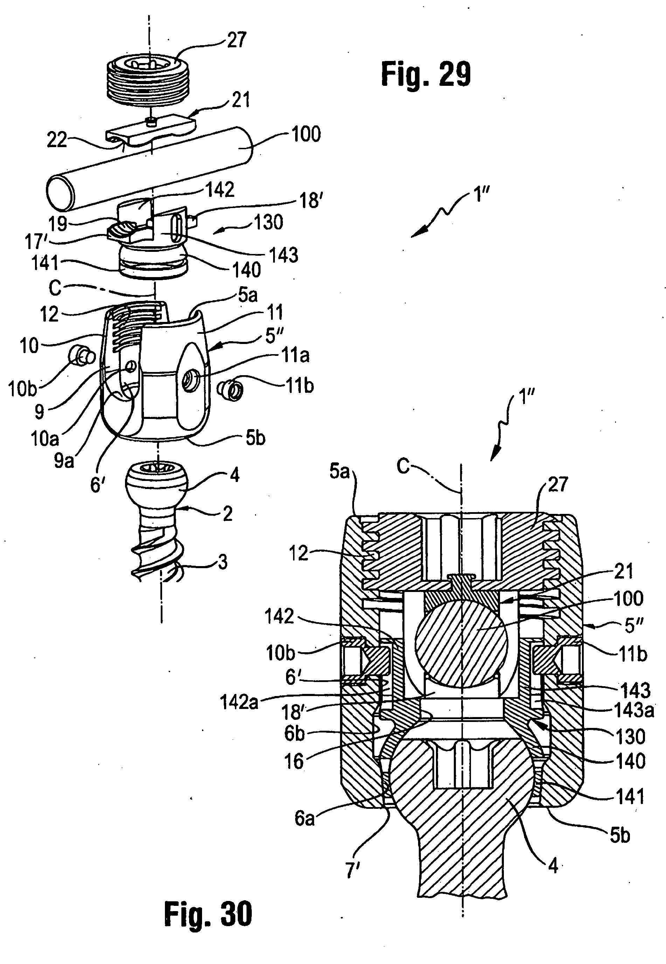

Referring to Figs. 29 and 30, a third embodiment of the polyaxial bone anchoring device is described. The polyaxial bone anchoring device according ot the third embodiment differs from the polyaxial bone anchoring device according to the first embodiment in that it is a bottom loading polyaxial bone anchoring device, i.e. the bone anchoring element 2 is configured to be introduced from the bottom end 5b of the receiving part. The receiving part 5" comprises a central coaxial bore 6' as in the first embodiment and an opening 7' at the second end 5b that has a diameter greater than the largest diameter of the head 4, so that the head 4 can be inserted from the second end 5b. Adjacent to the opening 7', there is a tapered section 6a that tapers towards the opening 7'. Between the tapered section 6a the central coaxial bore 6', comprises an enlarged bore section 6b with a greater diameter than that of the central bore 6'. The enlarged bore section serves for providing a space for the first clamping part 130 to expand therein. At about the center of each of the legs 10, 11 of the receiving part 5", there is a through hole 10a, 11a that extends transverse to the central axis C and accommodates a pin 10b, 11b, respectively. The pins 10b, 11b are configured to extend in the inserted state into the central coaxial bore 6'.

The first clamping part 130 is a monolithic piece. It comprises a first cap-like portion 140 which is flexible, for example by means of a slit ring portion 141 that is part of the cap-like portion. The flexibility can be achieved through various other structures, such as, for example a plurality of longitudinal and/or transverse slits. The size of the cap-like portion 140 is such that the head 4 of the bone anchoring element 2 can be inserted therein and is held therein in a frictional manner. As shown in Fig. 30, the cap-like portion 140 extends over the region with the largest diameter of the head 4. An outer surface ot the cap-like portion 140 at the lower end may be tapered and is configured to cooperate with the tapered section 6a of the receiving part 5"

The first clamping part 130 also comprises a coaxial bore 16 that allows access to the head 4 of the anchoring element with a tool. The first clamping part 130 further has two laterally extending arms 17', 18' that are offset from each other by 180° and extend from the bore 16 in a direction perpendicular to the bore axis beyond the outer surface of the cap-like portion 140. The arms 17', 18' have substantially the same shape as the arms 17, 18 of the first clamping part 13 of the first embodiment. The upper surface of the arms 17', 18' forms the rod supporting surface 19 as in the first embodiment.

At 90° offset from the arms 17', 18', there are two upstanding legs 142, 143 with a free end at a side opposite to the cap-like portion 140. At an outer surface of the upstanding legs 142, 143 a recess 142a, 143a is provided that is elongate in a direction parallel to the central axis C. The elongate recesses 142a, 143 a are configured to accommodate a front portion of the pins 10b, 11b. The bottom end of the elongate recesses 142a, 142b forms a stop for the first clamping part 130 when the first clamping part 130 is moved in the bore 6' towards the first end 5a of the receiving part 5" and the pins 10b, 11b abut against the bottom end of the recesses.

The second clamping part 21 is the same as in the first embodiment.

In use, the first clamping part 130 may be preassembled with the receiving part 5". The arms 17', 18' extend out of the bore 6' into the recess 9. The bone anchoring element 4 is inserted from the second end 5b into the receiving part. In the insertion position, the first clamping part is moved towards the first end 5a until the pins 10b, 11b abut against the bottom of the elongate recesses 142a, 142b. When the head 4 is inserted into the cap-like portion 140, the cap-like portion 140 expands into the enlarged bore section 6b of the receiving part 5". When the rod 100 is inserted, it rests on the arms 17', 18' of the first clamping part 130. Then the second clamping part 21 which is mounted to the locking screw 27 is inserted into the receiving part and the locking screw 27 is tightened. Thereby, the rod 100 is clamped between the arms 17', 18' and the second clamping part 21 and the pressure exerted onto the first clamping part 130 moves the first clamping part 130 downward towards the second end 5b. The cap-like portion 140 of the first clamping part 130 is gradually compressed in the tapered portion 6a of the receiving part 5" so that the head 4 is clamped in the cap-like portion 140 and prevented from falling out through the opening 7'. The final tightening of the locking screw 27 locks the bone anchoring element 2 in a specific angular position with respect to the central axis C. The effects on the rod and the rod surface that is clamped between the first and the second clamping part are the same as in the previous embodiments. Curved rods can also be used.

Many other modifications of the embodiments may be contemplated. For example, the bone anchoring devices shown in the embodiments are not limited to the use together with solid rods. There may be used also together with tubular rods. Also, flexible rods may be contemplated, for example elastomer rods.

Various modifications of the receiving part may be contemplated as well. For example, other designs of top loading or bottom loading polyaxial anchoring devices that comprise a pressure element for clamping the head can be used for the receiving part.
Other modifications of the locking device are possible. The locking device may include an outer nut or a cap. The connection of the locking element and the receiving part is not limited to a threaded connection.

The first clamping part has been described to include a portion 14 that acts onto the head 4 of the anchoring element. It may be contemplated that the portion acting onto the head is an element separate from the rod supporting portion. The length of the first clamping part and the second clamping part in the longitudinal direction of the U-shaped recess may be smaller than the length of the recess but should be greater than the diameter of the coaxial bore of the receiving part.

## Claims

1. Spinal stabilization system comprising
an anchoring element (2) comprising a shank (3) and a head (4);
a single rod (100, 101),
a receiving part (5, 5') comprising a first end (5a), an opposite second end (5b), a central axis (C) extending through the first end (5a) and the second end (5b), a coaxial bore (6), a seat (8) for the head (4) near the second end (5b) configured to pivotably receive the head (4) and a recess (9, 9') adjacent to the first end (5a) for receiving the single rod (100, 101), wherein two legs (10, 11) are formed by the recess (9, 9') and wherein a longitudinal axis (L) of the recess is substantially perpendicular to the central axis (C);
a first clamping part (13, 13') that is configured to support the single rod (100, 101) in the recess (9; 9');
a second clamping part (21, 21') that is configured to exert pressure onto the single rod (100, 101) when the single rod (100, 141) is in the recess, wherein the second clamping part (21, 21') extends laterally from of the coaxial bore (6) into the recess (9, 9') between the legs (10, 11);
a locking device (27) that is configured to exert pressure onto the second clamping part (21, 21') to secure the single rod (100, 101) in the recess (9, 9');
**characterized in that** the first clamping part (13, 13') has a rod supporting surface portion (19, 19') that extends laterally from of the coaxial bore (6) into the recess (9, 9') between the legs (10, 11).

2. The spinal stabilization system of claim 1, wherein the rod supporting surface portion (19, 19') forms a first groove (17, 18; 70) that has a longitudinal axis (L) perpendicular to the central axis (C).

3. The spinal stabilization system of claim 2, wherein the rod supporting surface portion (19, 19') comprises at least two projections (20a, 20b; 71a, 71 b) along the longitudinal axis of the first groove (17, 18; 70) that are separated by a depression.

4. The spinal stabilization system of one of claims 1 to 3, wherein the first clamping part (19, 19') comprises a coaxial bore (6) that is coaxial to the central axis (C).

5. The spinal stabilization system of claim 1 to 4, wherein the rod supporting surface (19) portion is comprised of two arms (17, 18) extending from the coaxial bore (6) of the receiving part into the recess (9, 9').

6. The spinal stabilization system of claim 5, wherein the first groove (70) has a portion (72) with reduced thickness in axial direction in such a way that it is flexible.

7. The spinal stabilization system of one of claims 1 to 6, wherein the first clamping part (13, 13') further comprises a cylindrical portion (14) that is configured to be movable in the coaxial bore (6) of the receiving part (5, 5') and that has on its side opposite to the rod contacting surface a recess (15) to cover at least a portion of the head (4).

8. The spinal stabilization system of one of claims 1 to 7, wherein the second clamping part (21, 21') has surface (22, 22') facing the single rod (100, 101) that forms a second groove having a longitudinal axis (L) perpendicular to the central axis (C).

9. The spinal stabilization system of claim 8, wherein the rod facing surface (22, 22') comprises at least two projections (24a, 24b; 90a, 90b) along the longitudinal axis of the second groove that are separated by a depression (23, 91).

10. The spinal stabilization system of one of claims 1 to 9, wherein the first clamping part (13, 13') and the second clamping part (21, 21') extend into the recess (9, 9') such that their free ends are substantially flush with the outer surface of the receiving part (5, 5').

11. The spinal stabilization system of one of claims 2 to 10, wherein the projections are bulges (20a, 20b) that are rounded in a longitudinal direction.

12. The spinal stabilization system of one of claims 2 to 11, wherein the projections (20a, 20b; 71a, 71b) of the first clamping part (13, 13') are at positions outside the coaxial bore (6) in a longitudinal direction of the recess (9, 9').

13. The spinal stabilization system of one of claims 8 to 12, wherein the projections (24a, 24b; 90a, 90b) of the second clamping part (21, 21') are at positions outside the coaxial bore (6) in a longitudinal direction of the recess (9, 9').

14. The spinal stabilization system according to one of claims 1 to 13, wherein the second clamping part is rotatably connected to the locking device.

15. The spinal stabilization system according to one of claims 1 to 14 further comprising at least one additional polyaxial bone anchoring device, wherein the single rod (100, 101) preferably comprises a NiTi alloy, in particular Nitinol and/or wherein the single rod (100, 101) preferably is a curved rod (101).

## Patentansprüche

1. Wirbelsäulenstabilisierungssystem, umfassend:
ein Verankerungselement (2) umfassend einen Schaft (3) und einen Kopf (4);
einen einzelnen Stab (100, 101),
ein Aufnahmeteil (5, 5') umfassend ein erstes Ende (5a), ein gegenüberliegendes zweites Ende (5b), eine sich durch das erste Ende (5a) und das zweite Ende (5b) erstreckende Mittenachse (C), eine koaxiale Bohrung (6), einen Sitz (8) für den Kopf (4) nahe dem zweiten Ende (5b), der eingerichtet ist, den Kopf (4) schwenkbar aufzunehmen, und eine Ausnehmung (9, 9') benachbart zu dem ersten Ende (5a) zum Aufnehmen des einzelnen Stabs (100, 101), wobei zwei Schenkel (10, 11) durch die Ausnehmung (9, 9') ausgebildet werden, und wobei eine Längsachse (L) der Ausnehmung im Wesentlichen senkrecht zur Mittenachse (C) ist;
ein erstes Klemmteil (13, 13'), das eingerichtet ist, den einzelnen Stab (100, 101) in der Ausnehmung (9; 9') zu halten;
ein zweites Klemmteil (21, 21'), das eingerichtet ist, Druck auf den einzelnen Stab (100, 101) auszuüben, wenn sich der einzelne Stab (100, 101) in der Ausnehmung befindet, wobei das zweite Klemmteil (21, 21') sich seitlich von der koaxialen Bohrung (6) in die Ausnehmung (9, 9') zwischen den Schenkeln (10, 11) erstreckt;
eine Verschlussvorrichtung (27), die eingerichtet ist, Druck auf das zweite Klemmteil (21, 21') auszuüben, um den einzelnen Stab (100, 101) in der Ausnehmung (9, 9') zu sichern;
**dadurch gekennzeichnet, dass** das erste Klemmteil (13, 13') einen Stabhalteoberflächenabschnitt (19, 19') besitzt, der sich seitlich von der koaxialen Bohrung (6) in die Ausnehmung (9, 9') zwischen den Schenkeln (10, 11) erstreckt.

2. Das Wirbelsäulenstabilisierungssystem gemäß Anspruch 1, wobei der Stabhalteoberflächenabschnitt (19, 19') eine erste Rinne (17, 18; 70) ausbildet, die eine Längsachse (L) senkrecht zur Mittenachse (C) besitzt.

3. Das Wirbelsäulenstabilisierungssystem gemäß Anspruch 2, wobei der Stabhalteoberflächenabschnitt (19, 19') wenigstens zwei Vorsprünge (20a, 20b; 71a, 71b) entlang der Längsachse der ersten Rinne (17, 18; 70) umfasst, die durch eine Vertiefung voneinander getrennt sind.

4. Das Wirbelsäulenstabilisierungssystem gemäß einem der Ansprüche 1 bis 3, wobei das erste Klemmteil (19, 19') eine koaxiale Bohrung (6) umfasst, die koaxial zur Mittenachse (C) ist.

5. Das Wirbelsäulenstabilisierungssystem gemäß Anspruch 1 bis 4, wobei der Stabhalteoberflächenabschnitt (19) zwei Arme (17, 18) umfasst, die sich von der koaxialen Bohrung (6) des Aufnahmeteils in die Ausnehmung (9, 9') erstrecken.

6. Das Wirbelsäulenstabilisierungssystem gemäß Anspruch 5, wobei die erste Rinne (70) einen Abschnitt (72) mit verminderter Dicke in axialer Richtung solchermaßen besitzt, dass er flexibel ist.

7. Das Wirbelsäulenstabilisierungssystem gemäß einem der Ansprüche 1 bis 6, wobei das erste Klemmteil (13, 13') ferner einen zylindrischen Abschnitt (14) umfasst, der eingerichtet ist, in der koaxialen Bohrung (6) des Aufnahmeteils (5, 5') beweglich zu sein, und welcher auf seiner Seite gegenüberliegend der Stabkontaktoberfläche eine Ausnehmung (15) besitzt, um wenigstens einen Abschnitt des Kopfs (4) zu umfassen.

8. Das Wirbelsäulenstabilisierungssystem gemäß einem der Ansprüche 1 bis 7, wobei das zweite Klemmteil (21,21') eine Oberfläche (22, 22') besitzt, die dem einzelnen Stab (100, 101) zugewandt ist, und die eine zweite Rinne mit einer Längsachse (L) senkrecht zur Mittenachse (C) ausbildet.

9. Das Wirbelsäulenstabilisierungssystem gemäß Anspruch 8, wobei die dem Stab zugewandte Oberfläche (22, 22') wenigstens zwei Vorsprünge (24a, 24b; 90a, 90b) entlang der Längsachse der zweiten Rinne umfasst, die voneinander durch eine Mulde (23, 91) getrennt sind.

10. Das Wirbelsäulenstabilisierungssystem gemäß einem der Ansprüche 1 bis 9, wobei sich das erste Klemmteil (13, 13') und das zweite Klemmteil (21, 21') in die Ausnehmung (9, 9') erstrecken, so dass deren freie Enden im Wesentlichen mit der äußeren Oberfläche des Aufnahmeteils (5, 5') flach abschließen.

11. Das Wirbelsäulenstabilisierungssystem gemäß einem der Ansprüche 2 bis 10, wobei die Vorsprünge Ausbauchungen (20a, 20b) sind, die in einer Längsrichtung gerundet sind.

12. Das Wirbelsäulenstabilisierungssystem gemäß einem der Ansprüche 2 bis 11, wobei sich die Vorsprünge (20a, 20b; 71a, 71b) des ersten Klemmteils (13, 13') an Positionen außerhalb der koaxialen Bohrung (6) in einer Längsrichtung der Ausnehmung (9, 9') befinden.

13. Das Wirbelsäulenstabilisierungssystem gemäß einem der Ansprüche 8 bis 12, wobei sich die Vorsprünge (24a, 24b; 90a, 90b) des zweiten Klemmteils (21, 21') an Positionen außerhalb der koaxialen Bohrung (6) in einer Längsrichtung der Ausnehmung (9, 9') befinden.

14. Das Wirbelsäulenstabilisierungssystem gemäß einem der Ansprüche 1 bis 13, wobei das zweite Klemmteil drehbar mit der Verschlussvorrichtung verbunden ist.

15. Das Wirbelsäulenstabilisierungssystem gemäß einem der Ansprüche 1 bis 14, ferner umfassend wenigstens eine zusätzliche polyaxiale Knochenverankerungsvorrichtung, wobei der einzelne Stab (100, 101) vorzugsweise eine NiTi-Legierung umfasst, insbesondere Nitinol, und/oder wobei der einzelne Stab (100, 101) vorzugsweise ein gekrümmter Stab (101) ist.

## Revendications

1. Système de stabilisation spinal, comprenant:
un élément d'ancrage (2) comprenant une mèche (3) et une tête (4);
une tige unique (100, 101),
une partie de réception (5, 5') comprenant une première extrémité (5a), une deuxième extrémité opposée (5b), un axe central (C) qui s'étend à travers la première extrémité (5a) et la deuxième extrémité (5b), un alésage coaxial (6), un siège (8) pour la tête (4) à proximité de la deuxième extrémité (5b) configuré de manière à recevoir de façon pivotante la tête (4) et un évidement (9, 9') adjacent à la première extrémité (5a) destiné à recevoir la tige unique (100, 101), dans lequel deux branches (10, 11) sont formées par l'évidement (9, 9'), et dans lequel un axe longitudinal (L) de l'évidement est sensiblement perpendiculaire à l'axe central (C);
une première partie de serrage (13, 13') qui est configurée de manière à supporter la tige unique (100, 101) dans l'évidement (9, 9');
une deuxième partie de serrage (21, 21') qui est configurée de manière à exercer une pression sur la tige unique (100, 101) lorsque la tige unique (100, 101) se trouve dans l'évidement, dans lequel la deuxième partie de serrage (21, 21') s'étend latéralement à partir de l'alésage coaxial (6) dans l'évidement (9, 9') entre les branches (10, 11);
un dispositif de verrouillage (27) qui est configuré de manière à exercer une pression sur la deuxième partie de serrage (21, 21') afin de fixer la tige unique (100, 101) dans l'évidement (9, 9'),
**caractérisé en ce que** la première partie de serrage (13, 13') présente une partie de surface de support de tige (19, 19') qui s'étend latéralement à partir de l'alésage coaxial (6) dans l'évidement (9, 9') entre les branches (10, 11).

2. Système de stabilisation spinal selon la revendication 1, dans lequel la partie de surface de support de tige (19, 19') forme une première rainure (17, 18; 70) présentant un axe longitudinal (L) perpendiculaire à l'axe central (C).

3. Système de stabilisation spinal selon la revendication 2, dans lequel la partie de surface de support de tige (19, 19') comporte au moins deux saillies (20a, 20b; 71a, 71b) le long de l'axe longitudinal de la première rainure (17, 18; 70) qui sont séparées par une dépression.

4. Système de stabilisation spinal selon l'une quelconque des revendications 1 à 3, dans lequel la première partie de serrage (19, 19') comprend un alésage coaxial (6) qui est coaxial à l'axe central (C).

5. Système de stabilisation spinal selon l'une quelconque des revendications 1 à 4, dans lequel la partie de surface de support de tige (19) est constituée de deux bras (17, 18) qui s'étendent à partir de l'alésage coaxial (6) de la partie de réception dans l'évidement (9, 9').

6. Système de stabilisation spinal selon la revendication 5, dans lequel la première rainure (70) comprend une partie (72) qui présente une épaisseur réduite dans la direction axiale de manière à être flexible.

7. Système de stabilisation spinal selon l'une quelconque des revendications 1 à 6, dans lequel la première partie de serrage (13, 13') comprend en outre une partie cylindrique (14) qui est configurée de manière à être mobile dans l'alésage coaxial (6) de la partie de réception (5, 5') et qui présente sur son côté opposé à la surface de contact de tige un évidement (15) destiné à couvrir au moins une partie de la tête (4).

8. Système de stabilisation spinal selon l'une quelconque des revendications 1 à 7, dans lequel la deuxième partie de serrage (21, 21') présente une surface (22, 22') opposée à la tige unique (100, 101) qui forme une deuxième rainure présentant un axe longitudinal (L) perpendiculaire à l'axe central (C).

9. Système de stabilisation spinal selon la revendication 8, dans lequel la surface opposée à la tige (22, 22') comporte au moins deux saillies (24a, 24b; 90a, 90b) le long de l'axe longitudinal de la deuxième rainure qui sont séparées par une dépression (23, 91).

10. Système de stabilisation spinal selon l'une quelconque des revendications 1 à 9, dans lequel la première partie de serrage (13, 13') et la deuxième partie de serrage (21, 21') s'étendent dans l'évidement (9, 9') de telle sorte que leurs extrémités libres soient sensiblement à fleur de la surface extérieure de la partie de réception (5, 5').

11. Système de stabilisation spinal selon l'une quelconque des revendications 2 à 10, dans lequel les saillies sont des bosses (20a, 20b) qui sont arrondies dans une direction longitudinale.

12. Système de stabilisation spinal selon l'une quelconque des revendications 2 à 11, dans lequel les saillies (20a, 20b; 71a, 71b) de la première partie de serrage (13, 13') sont situées à des positions extérieures à l'alésage coaxial (6) dans une direction longitudinale de l'évidement (9, 9').

13. Système de stabilisation spinal selon l'une quelconque des revendications 8 à 12, dans lequel les saillies (24a, 24b; 90a, 90b) de la deuxième partie de serrage (21, 21') sont situées à des positions extérieures à l'alésage coaxial (6) dans une direction longitudinale de l'évidement (9, 9').

14. Système de stabilisation spinal selon l'une quelconque des revendications 1 à 13, dans lequel la deuxième partie de serrage est connectée de façon rotative au dispositif de verrouillage.

15. Système de stabilisation spinal selon l'une quelconque des revendications 1 à 14, comprenant en outre au moins un dispositif d'ancrage d'os polyaxial supplémentaire, dans lequel la tige unique (100, 101) comprend de préférence un alliage de NiTi, en particulier de Nitinol et/ou dans lequel la tige unique (100, 101) est de préférence une tige courbe (101).
